# EUROPÄISCHE PATENTSCHRIFT

(11) **EP 2 061 532 B1**
(45) Veröffentlichungstag und Bekanntmachung des Hinweises auf die Patenterteilung: **12.05.2010**
(21) Anmeldenummer: 07820129.0
(22) Anmeldetag: 11.09.2007
(51) Int. Cl.: A61M 1/34, A61M 1/36, A61M 1/16

(54) **THERAPIEEINRICHTUNG MIT GEDÄCHTNISGESTÜTZTER REGELEINRICHTUNG**
TREATMENT DEVICE WITH MEMORY-SUPPORTED CONTROL MEANS
DISPOSITIF THÉRAPEUTIQUE AVEC SYSTÈME DE RÉGULATION À MÉMOIRE

(30) Priorität: 15.09.2006 EP 06120761
(43) Veröffentlichungstag der Anmeldung: 27.05.2009
(73) Patentinhaber: B. Braun Avitum AG, 34212 Melsungen (DE)
(72) Erfinder: RÖHER, Otfried, 01139 Dresden (DE); KORTH, Steffen, 99334 Elleben (DE)
(74) Vertreter: von Kreisler Selting Werner
(86) Internationale Anmeldenummer: PCT/EP2007/059524
(87) Internationale Veröffentlichungsnummer: WO 2008/031821

(56) Entgegenhaltungen:
- EP-A2- 0 956 872
- EP-A2- 1 226 838
- DE-A1- 2 825 134
- US-A- 4 710 164
- US-A1- 2003 209 475

## Beschreibung

Die Erfindung betrifft eine Therapieeinrichtung mit einem Blutdruckmessgerät und einer gedächtnisgestützten Regeleinrichtung zur Steuerung der Zeitpunkte der Blutdruckmessungen und zur Regelung des Blutdruckes des Patienten während eines Therapievorganges, insbesondere ein Therapiegerät zur extrakorporalen Blutreinigung.

Bei verschiedenen Therapien, wie beispielsweise bei der Hämodialyse, ist es erforderlich, den Blutdruck des Patienten laufend zu überwachen. Hierfür werden Geräte zur unblutigen (non-invasiven) Blutdruckmessung mit einer um den Arm des Patienten herumlegbaren Manschette benutzt, die aufgepumpt und dann unter Druckmessung langsam entlastet wird. Um eine quasikontinuierliche Blutdruckregelung zu erreichen, muss die Regeleinrichtung in Intervallen von wenigen Minuten Regelvorgänge durchführen. Die während einer mehrstündigen Behandlung hierfür erforderliche Anzahl von Blutdruckmessungen kann erheblich reduziert werden, wenn zu festgelegten Zeitpunkten die Blutdruckmessungen durch Blutdruckwerte substituiert werden, die aus früheren Behandlungen desselben Patienten gewonnen wurden.

In U.S. 6,579 ,241 B2 und EP 1 226 838 A2 sind entsprechende Therapieeinrichtungen mit intervallartiger Blutdruckmessung und mit reduzierter Anzahl der Messvorgänge beschrieben. Anhand der gleichen Länge aller Intervalle, beispielsweise 5 Minuten je Intervall, gewährleistet hierbei die Regeleinrichtung die Auswertung des Blutdruckverlaufes und der Blutdrucktrends in jedem Intervall nach einheitlichen Regeln, wie dies in EP 0 956 872 A2 beschrieben ist.

EP 1 226 838 A2 beschreibt eine Therapieeinrichtung, die Blutdruckmessungen in Intervallen ausführt und in Abhängigkeit hiervon die Ultrafiltrationsrate UFR in der Weisel, vegelt dass keine unzulässigen Blutdruckabfälle auftreten. Zu den Zeitpunkten, in denen keine Messung des Blutdrucks durchgeführt wird, werden die Werte einer Leitkurve, die aus früheren Therapieabläufen ermittelt wurde, als Ist-Werte des Blutdrucks zugrundegelegt, und in den Zeitpunkten der Blutdruckmessung werden die aktuellen Werte des Blutdrucksverlaufs ausgewertet.

Da für morbide Ereignisse während der Hämodialyse, wie Hypotonie, Schwindelanfälle, Erbrechen u. a., multifaktorielle Ursachen zu berücksichtigen sind, werden in der klinischen Praxis mitunter Monitore für weitere Größen (beispielsweise EKG, relatives Blutvolumen, Hämatokrit, Blutsauerstoffgehalt) zur Erkennung von Komplikationen verwendet. Hieraus können sich in Abhängigkeit von den Messergebnissen zu beliebigen Zeitpunkten Anforderungen für zusätzliche Blutdruckmessungen ergeben. Klinische Erfahrungen mit Dialysepatienten zeigen, dass in Abhängigkeit vom Zustand des Patienten mitunter auch Blutdruckmessungen direkt vom medizinischen Personal angefordert werden. Eine entsprechende Therapieeinrichtung mit zeitflexibler Blutdruckregelung ist in der (nicht vorveröffentlichten) EP1844800 beschrieben.

Die genannten Therapieeinrichtungen nutzen für die Blutdruckregelung grundsätzlich nur Werte und Zeitverläufe der vorangegangenen Therapievorgänge bis zum gegenwärtigen Behandlungszeitpunkt. Die in den Therapieeinrichtungen ebenfalls gespeicherten Werte und Zeitverläufe der vorangegangenen Therapievorgänge für die Zeitabschnitte nach dem gegenwärtigen Behandlungszeitpunkt bis zum Ende der Behandlung werden nicht in die Blutdruckregelung einbezogen.

Klinische Erfahrungen mit Dialysepatienten zeigen jedoch, dass sich aus diesen Zeitabschnitten vorangegangener Therapievorgänge wesentliche Informationen über den zu erwartenden Blutdruckverlauf nach dem jeweiligen Behandlungszeitpunkt der gegenwärtigen Behandlung ableiten lassen. Es erscheint aussichtsreich, dass sich daraus insbesondere für den selben Patienten bei vergleichbaren Behandlungsparametern, wie Gesamt-Ultrafiltrationsvolumen, maximale Ultrafiltrationsrate, Dialysatleitfähigkeit, Behandlungsdauer u.a., wichtige Schlussfolgerungen ergeben, die in der klinischen Praxis routinemäßig für eine vorausschauende Blutdruckregelung genutzt werden können. In Verbindung mit analytischen Vergleichen der Zeitverläufe des Blutdruckes sowie weiterer relevanter Einflussgrößen während der gegenwärtigen Behandlung und während vorangegangener Therapievorgänge anhand statistischer Ähnlichkeitskriterien ergeben sich günstige Voraussetzungen für die Voraussage bevorstehender Blutdruckabfälle.

Der Erfindung liegt deshalb die Aufgabe zu Grunde, eine Therapieeinrichtung derart auszubilden, dass mit Hilfe einer gedächtnisgestützten Regeleinrichtung eine Voraussage über den bevorstehenden Blutdruckverlauf getroffen wird und eine vorausschauende Blutdruckregelung erfolgt.

Die Lösung dieser Aufgabe erfolgt erfindungsgemäß mit den Merkmalen des Patentanspruches 1. Hiernach ist die gedächtnisgestützte Regeleinrichtung zum Ausführen folgender weiterer Funktionen nach einer Blutdruckmessung ausgebildet:
c) Analysieren der Verläufe des Blutdruckes der ausgewählten vorangegangenen Therapievorgänge in auf den aktuellen Zeitpunkt des gegenwärtigen Therapievorganges nachfolgenden Zeitabschnitten auf Blutdruckabfälle und Selektieren mindestens eines der vorangegangenen Therapievorgänge mit Blutdruckabfall in den nachfolgenden Zeitabschnitten;
d) Vorausschauende Blutdruckregelung zur Prävention von Blutdruckabfällen, wobei der Zeitverlauf des mindestens einen selektierten Therapievorganges als Führungsgröße der vorausschauenden Blutdruckregelung in den auf den aktuellen Zeitpunkt nachfolgenden Zeitabschnitten verwendet wird.

Erfindungsgemäß werden die Verläufe des Blutdruckes vorangegangener Therapievorgänge in auf den aktuellen Zeitpunkt des gegenwärtigen Therapievorganges nachfolgenden Zeitabschnitten auf Blutdruckabfälle untersucht. Auf diese Weise werden diejenigen vorangegangenen Therapievorgänge, bei denen in einem Zeitraum nach dem aktuellen Zeitpunkt des gegenwärtigen Therapievorganges mindestens ein Blutdruckabfall auftrat, selektiert und für die Regelung werden nur die Verläufe bei den selektierten Therapievorgängen benutzt. Durch die Einbeziehung der Zeitverläufe des Blutdruckes (und eventuell weiterer relevanter Einflussgrößen) aus vorangegangenen Therapievorgängen werden eine Voraussage bevorstehender Blutdruckabfälle und eine vorausschauende Blutdruckregelung auf der Grundlage patientenspezifischer Erfahrungen ermöglicht. Unter vergleichbaren Therapiebedingungen bezüglich der vom medizinischen Personal vorgegebenen Behandlungsmerkmale, wie Gesamt-Ultrafiltrationsvolumen, Therapiedauer, maximale Ultrafiltrationsrate, Dialysatleitfähigkeit und Grenzwerte des Blutdruck-Regelbereiches, wurden bei der Voraussage von bevorstehenden Abnormalitäten, wie Blutdruckabfällen, innerhalb des nachfolgenden Zeitabschnittes von z. B. 30 Minuten Trefferraten von über 85% erzielt.

Mit dem Merkmal d) wird erreicht, dass die auf den aktuellen Zeitpunkt folgende Blutdruckregelung eine "vorausschauende Blutdruckregelung" ist, die sich an einem selektierten Zeitverlauf als Führungsgröße orientiert.

Da das Kriterium "Blutdruckabfall" bereits bei der Selektion berücksichtigt wird, erfolgt die Regelung des Blutdrucks in einer Weise, dass zukünftige Blutdruckabfälle mit hoher Wahrscheinlichkeit vermieden werden.

Die Erfindung leistet insbesondere bei hypotonie-gefährdeten Patienten einen wesentlichen Beitrag zur Prävention von Blutdruckabfällen und damit zur Qualitätsverbesserung der Dialysebehandlung.

Vorzugsweise führt die gedächtnisgestützte Regeleinrichtung, die unter anderem auch die intervallartigen Blutdruckmessungen steuert, die folgenden Schritte aus:
- Speicherung der Blutdruckmesswerte für eine festgelegte Anzahl von Therapievorgängen, einschließlich von zu festgelegten Zeitpunkten aus vorangegangenen Therapievorgängen in Form von Leitkurven übernommenen Substitutionswerten und der Führungsgrößen der gedächtnisgestützten Blutdruckregelung (zum Beispiel Ultrafiltrationsrate, Dialysatleitfähigkeit),
- Speicherung weiterer Behandlungsparameter (zum Beispiel Gesamt-Ultrafiltrationsvolumen, maximale Ultrafiltrationsrate, Therapiedauer, Grenzwerte des Blutdruck-Regelbereiches) für die gemäß d) gespeicherten Therapievorgänge,
- Analyse und Klassierung der unter d) genannten Blutdruckwerte für verschiedene Zeitabschnitte der vorangegangenen Therapievorgänge entsprechend ihrer Ähnlichkeit zum Blutdruckverlauf des gegenwärtigen Therapievorganges,
- Ermittlung von Maßnahmen zur vorausschauenden Blutdruckregelung für die verwendeten Führungsgrößen anhand derjenigen vorangegangenen Therapievorgänge mit der größten Ähnlichkeit zum gegenwärtigen Blutdruckverlauf,
- Anpassung der Regelcharakteristiken entsprechend den bei dem Schritt Ermittlung ermittelten Maßnahmen in Abhängigkeit vom gegenwärtigen Blutdruckverlauf und vom Zeitpunkt während des laufenden Therapievorganges.

Von der gedächtnisgestützten Regeleinrichtung werden die folgenden Kategorien von Blutdruckwerten in die vorausschauende Blutdruckregelung einbezogen:
- Blutdruckwerte, die durch intervallartige Messungen gemäß dem in der Regeleinrichtung implementierten Zeitregime ermittelt werden, wie dies in EP 0 956 872 B1 beschrieben ist, und
- Blutdruckwerte, die zwecks Reduzierung der Anzahl der Blutdruckmessungen in Form von Leitkurven aus vorangegangenen Therapievorgängen substituiert werden, wie dies in der Europäischen Patentanmeldung EP 1 226 838 A2 beschrieben ist, und
- Blutdruckwerte, die von angeschlossenen Mess- und Auswertegeräten für andere physiologische Größen (zum Beispiel EKG, Relatives Blutvolumen, Hämatokrit, Blutsauerstoffgehalt) zu anderen Zeitpunkten automatisch angefordert werden, wie dies in der Europäischen Patentanmeldung 06 112 431.9 beschrieben ist, und
- Blutdruckwerte, die vom medizinischen Personal manuell zu anderen Zeitpunkten angefordert werden, wie dies in der Europäischen Patentanmeldung 06 112 431.9 beschrieben ist.

Gemäß einer bevorzugten Weiterbildung der Erfindung ist vorgesehen, dass die gedächtnisgestützte Regeleinrichtung nach jeder Blutdruckmessung alle gespeicherten Therapieverläufe des jeweiligen Patienten hinsichtlich ihrer Ähnlichkeit zum gegenwärtigen Blutdruckverlauf analysiert. Um stets einen aktuellen Daten-Pool zu gewährleisten, ist die Speicherung von maximal 100 Behandlungen je Patient vorgesehen (n = 100). Beim üblichen Behandlungsrhythmus von drei Dialysen je Woche repräsentiert der gespeicherte Daten-Pool die Behandlungen von bis zu 33 Wochen und somit maximal etwa acht Monate. Bei Speicherung der 101. Behandlung wird die 1. Behandlung automatisch gelöscht. Dadurch ist eine laufende Aktualisierung des Daten-Pools gewährleistet.

Gemäß einer bevorzugten Weiterbildung der Erfindung ist weiterhin vorgesehen, dass nach jeder Blutdruckmessung für die vorausschauende Blutdruckregelung eine bestimmte Anzahl von z. B. 3 bis 5 vorangegangenen Behandlungen mit der jeweils größten Ähnlichkeit zum gegenwärtigen Blutdruckverlauf analysiert werden. Dadurch wird gewährleistet, dass im Interesse einer möglichst hohen Voraussagewahrscheinlichkeit bevorstehender Blutdruckabfälle nur Behandlungen betrachtet werden, deren Ähnlichkeit sich markant von der Mehrzahl der gespeicherten Behandlungen abhebt. Die Ähnlichkeitsanalysen erfassen vorzugsweise die Zeitabschnitte vom Beginn der Behandlung bis zum gegenwärtigen Zeitpunkt oder nur 15 bis 20 Minuten vor dem gegenwärtigen Zeitpunkt oder eine Kombination beider Varianten.

Gemäß einer bevorzugten Weiterbildung der Erfindung ist weiterhin vorgesehen, dass die vorausschauende Blutdruckregelung aktiviert wird, sobald die Mehrzahl der analysierten 3 bis 5 vorangegangenen Therapievorgänge im nachfolgenden Zeitabschnitt von etwa 30 Minuten Blutdruckwerte innerhalb des aktiven Blutdruck-Regelbereiches aufweisen. Die gedächtnisgestützte Regeleinrichtung reagiert dann mit einer Reduzierung der Ultrafiltrationsrate und/oder Erhöhung der Dialysatleitfähigkeit.

Die Erfindung eignet sich insbesondere für Dialysegeräte und andere Geräte für die extrakorporale Blutreinigung, jedoch auch beispielsweise für die Infusionstherapie.

Im Folgenden wird unter Bezugnahme auf die Zeichnungen ein Ausführungsbeispiel der Erfindung näher erläutert.

Es zeigen:
- Fig. 1: eine schematische Darstellung eines Dialysegerätes,
- Fig. 2: ein Zeitdiagramm einer Dialysebehandlung (Auszug) ohne vorausschauende Blutdruckregelung, und
- Fig. 3: ein Zeitdiagramm einer Dialysebehandlung (Auszug) mit vorausschauender Blutdruckregelung.

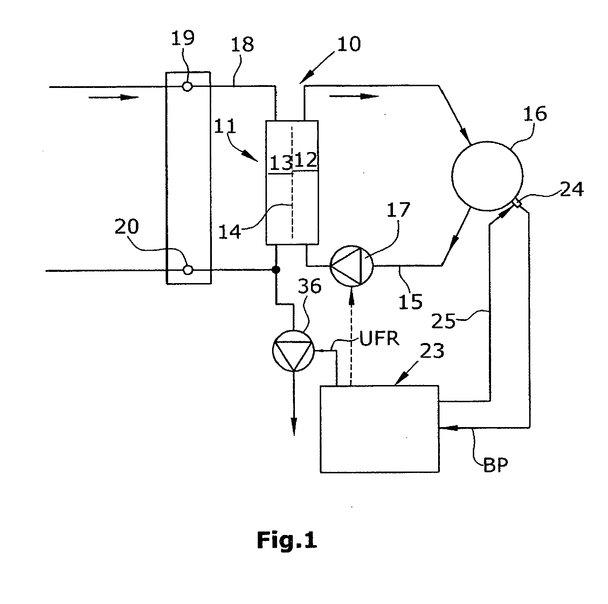

Das in Fig. 1 dargestellte Dialysegerät entspricht in seinem Grundaufbau dem von EP 0 956 872 B1 oder von EP 1 226 838 A2. Das Dialysegerät 10 enthält eine Ultrafiltrationsvorrichtung 11 mit einer Primärkammer 12 und einer Sekundärkammer 13, die durch eine Membran 14 voneinander getrennt sind. Die Primärkammer 12 ist Bestandteil eines Blutkreislaufes 15, bei dem Blut, welches über das arterielle System des Patienten entnommen wurde, in der Ultrafiltrationsvorrichtung 11 gereinigt und anschließend über das venöse System zum Patienten 16 zurückgeführt wird. In dem Blutkreislauf 15 befindet sich eine Pumpe 17. Diese ist als volumetrische Pumpe ausgebildet, d. h. die Fördermenge dieser Pumpe entspricht ihrer Antriebsgeschwindigkeit, und ist steuerbar.

Die Sekundärkammer 13 der Ultrafiltrationsvorrichtung 11 befindet sich in einem Dialysierflüssigkeitsweg 18, in dem Dialysierflüssigkeit gepumpt wird. Die Dialysierflüssigkeit kommt von einem (nicht dargestellten) Vorratsbehälter, nimmt in der Ultrafiltrationsvorrichtung 11 zusätzliche Substanzen aus dem Blut auf und wird dann zu einem (nicht dargestellten) Ablauf gepumpt. In dem Dialysierflüssigkeitsweg ist vor und hinter der Sekundärkammer 13 jeweils eine Durchflusskammer 19 bzw. 20 angeordnet, welche die Durchflussrate an der betreffenden Stelle regelt. Die Durchflusskammer 19 und die Durchflusskammer 20 haben die gleiche Förderrate. Über die volumengesteuerte Ultrafiltrationspumpe 36 wird die gewünschte Ultrafiltrationsmenge UFV in einer festgelegten Ultrafiltrationsrate UFR abgezogen. Das Zeitintegral über die Ultrafiltrationsrate UFR bildet das Ultrafiltrationsvolumen UFV, also dasjenige Flüssigkeitsvolumen, das seit Beginn der Behandlung die Membran 14 passiert hat. Die Regelung der Ultrafiltrationsrate erfolgt durch die Regeleinrichtung 23, die Steuersignale für die Förderrate der Pumpe 36 liefert. Die Förderrate der Pumpe wird in der Weise eingestellt, dass sich eine gewünschte Ultrafiltrationsrate ergibt.

Die Regeleinrichtung 23 empfängt ferner das Blutdrucksignal BP eines Blutdruckmessers 24, der am Körper des Patienten angebracht ist. Der Blutdruckmesser weist eine aufblasbare Manschette auf, die um den Arm des Patienten gelegt ist, und führt nicht-invasive Blutdruckmessungen aus. Die Zeitpunkte der Blutdruckmessungen werden intern durch die Regeleinrichtung 23 über die Leitungen 25 und extern durch angeschlossene Monitore und/oder das medizinische Personal festgelegt. Neben dem Blutdruckwert können der Regeleinrichtung 23 auch noch Blutdrucktrendwerte zugeführt werden wie es in EP 0 956 872 B1 beschrieben ist. Die Regeleinrichtung 23 regelt in Abhängigkeit von den erhaltenen Eingangsgrößen die Ultrafiltrationsrate UFR.

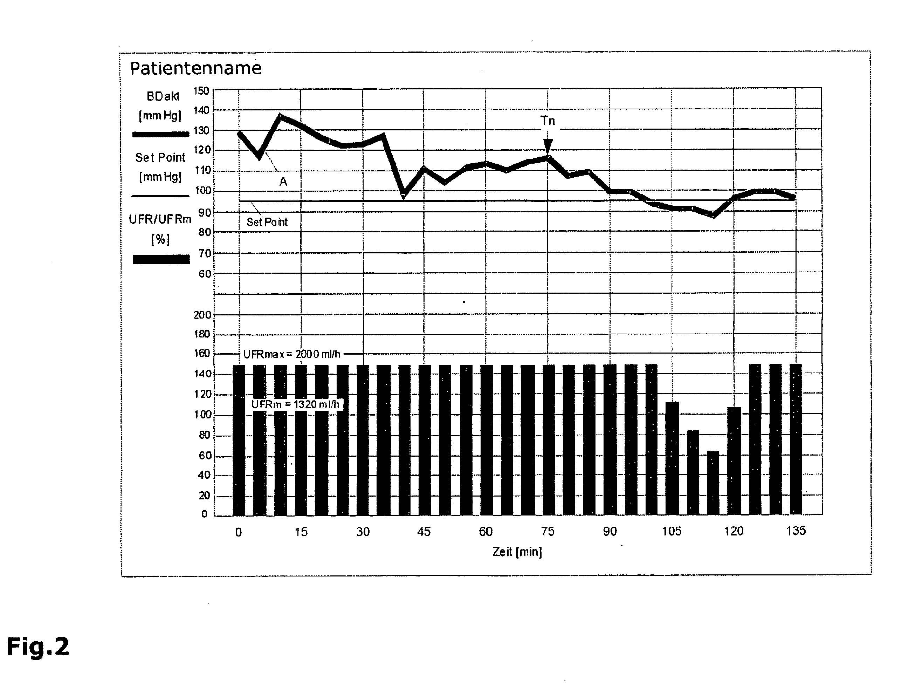

Fig. 2 veranschaulicht am Beispiel einer Dialysebehandlung die Blutdruckregelung, wie sie von der Regeleinrichtung 23 bisher ohne vorausschauende Blutdruckregelung gesteuert wird. Zu dem betrachteten Behandlungszeitpunkt Tn = 75 Minuten ergibt sich aus der hier durchgeführten Blutdruckmessung ein Blutdruck, der beträchtlich über dem oberen Grenzwert des aktiven Regelbereiches der Regeleinrichtung liegt. Dieser obere Grenzwert ist in Fig. 2 als Set Point eingezeichnet. Durch die Regeleinrichtung erfolgt deshalb anhand des Verlaufes der Blutdruckkurve A keine Reduzierung der Ultrafiltrationsrate, so dass die vom medizinischen Personal vorgewählte maximale Ultrafiltrationsrate UFRmax = 2000 ml/h aufrecht erhalten wird. UFRmax beträgt in diesem Beispiel etwa 150% der mittleren Ultrafiltrationsrate UFRm = 1320 ml/h, die sich aus dem vorgegebenen Dialyseziel (Gesamt-Ultrafiltrationsvolumen und Behandlungsdauer) ergibt. Der zu einem späteren Zeitpunkt bei 100 Minuten auftretende Blutdruckabfall unter den Set Point wird von der bisher verwendeten Regeleinrichtung ohne vorausschauende Blutdruckregelung nicht wahrgenommen, da von ihr gespeicherte Blutdruckverläufe vorangegangener Behandlungen nur bis zum jeweiligen Behandlungszeitpunkt (in Fig. 2: Tn = 75 min) berücksichtigt werden. Die Regeleinrichtung beginnt deshalb in diesem Beispiel erst zum Zeitpunkt der Unterschreitung des Set Point, d. h. erst nach 100 min, mit der Reduzierung der Ultrafiltrationsrate.

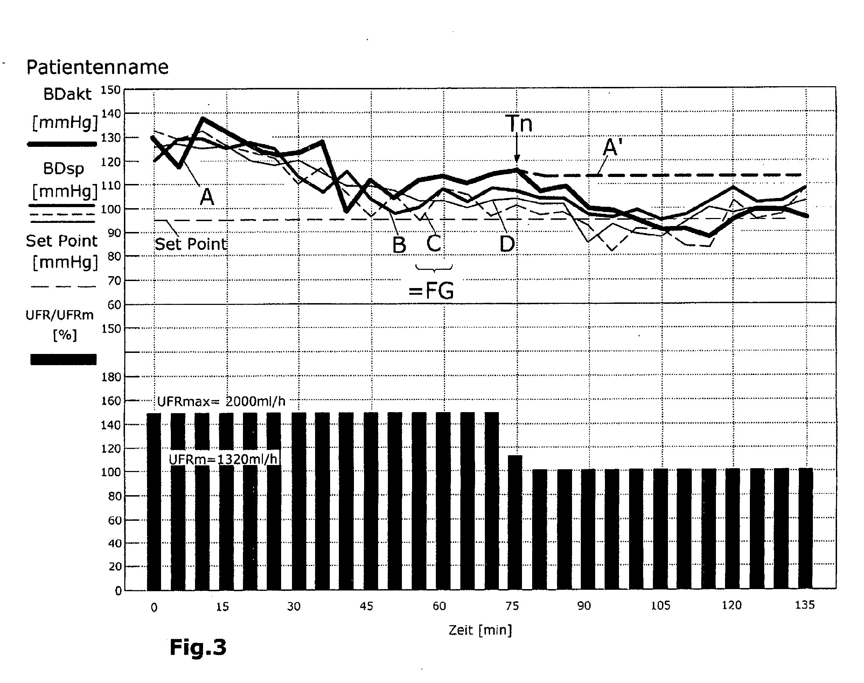

Fig. 3 veranschaulicht die Vorteile einer erfindungsgemäßen Regeleinrichtung mit vorausschauender Blutdruckregelung anhand des selben Blutdruckverlaufes A bis zum betrachteten Behandlungszeitpunkt Tn = 75 min wie in Fig. 2. Zusätzlich sind in Fig. 3 die gespeicherten Blutdruckwerte BDsp der drei Blutdruckkurven B, C und D aus vorangegangenen Behandlungen eingezeichnet, die anhand der in der Regeleinrichtung implementierten Ähnlichkeitskriterien für den gegenwärtigen Behandlungsabschnitt von 0 min bis 75 min die größte Ähnlichkeit zum aktuellen Blutdruckverlauf BDakt aufweisen. Die Ähnlichkeitskriterien ergeben sich hierbei aus statistischen Analysen der Blutdruckverläufe (beispielsweise Mittelwerte, Standardabweichungen, t-Tests, Mustererkennung).

Die gedächtnisgestützte Regeleinrichtung mit vorausschauender Blutdruckregelung erkennt jetzt bereits zum betrachteten Behandlungszeitpunkt Tn = 75 min, dass zwei der drei selektierten Blutdruckkurven aus vorangegangen Behandlungen im gewählten Voraussagezeitraum Tn+30min, d. h. im nachfolgenden Behandlungsabschnitt von 75 min bis 105 min, Blutdruckabfälle unter den Set Point, aufweisen, nämlich die Kurven C und D.

Da zwischen einer Änderung der Ultrafiltrationsrate und der zugehörigen Änderung des Blutdruckes physiologische Reaktionszeiten von mehreren Minuten zu berücksichtigen sind, werden für die vorausschauende Blutdruckregelung deshalb die gespeicherten Blutdruckverläufe BDsp der selektierten vorangegangenen Therapievorgänge im Zeitabschnitt von Tn + 15 min bis Tn + 30 min analysiert. Für die vorausschauende Blutdruckregelung sind hierbei sowohl die Zeitpunkte wie auch die Tiefe der Blutdruckabfälle wesentlich. Im gezeigten Beispiel Fig. 3 liegen für die Kurven C und D die Zeitpunkte des Blutdruckabfalls unter den Set Point bei 90 min. Die Tiefe des Blutdruckabfalls ist jedoch für Kurve C im Zeitabschnitt von 90 min bis 105 min wesentlich größer als für Kurve D. Deshalb selektiert die Regeleinrichtung in diesem Beispiel den Blutdruckverlauf der Kurve C ab Tn + 15 min = 90 min und benutzt ihn als Führungsgröße FG der vorausschauenden Blutdruckregelung für den Behandlungsabschnitt von Tn = 75 min bis zur nächsten Blutdruckmessung.

Die Regeleinrichtung veranlasst deshalb jetzt bereits bei Tn = 75 min eine vorausschauende Reduzierung der Ultrafiltrationsrate mit dem Ziel, dadurch einen Blutdruckabfall unter den Set Point wie bei Kurve A in der gegenwärtigen Behandlung möglichst zu vermeiden oder zumindest zu minimieren. Diese Zielfunktion ist in Fig. 3 durch den zu erwartenden hypothetischen Blutdruckverlauf A' angedeutet. Wesentliche Einflussgrößen der gegenwärtigen Behandlung und der selektierten Blutdruckverläufe B, C und D, wie beispielsweise Gesamtultrafiltrationsvolumen, Therapiedauer, maximale Ultrafiltrationsrate, oberer und unterer Grenzwert des Blutdruckregelbereiches, werden bei der Bemessung der jeweiligen Reduzierung der Ultrafiltrationsrate berücksichtigt.

Die gedächtnisgestützte Regeleinrichtung mit vorausschauender Blutdruckregelung wiederholt die in Fig.3 veranschaulichten Arbeitsschritte nach jeder Blutdruckmessung. Hierfür sind im implementierten Zeitregime in Abhängigkeit von der laufenden Behandlungszeit und dem bereits entzogenen Ultrafiltrationsvolumen Zeitabschnitte von 15min bis 30min festgelegt. Der tatsächliche Verlauf von A' während der laufenden Behandlung wird deshalb durch jede nachfolgende Blutdruckmessung im Sinne einer schrittweisen Optimierung der Behandlung modifiziert.

## Patentansprüche

1. Therapieeinrichtung für eine extrakorporale Blutbehandlung mit einstellbaren Zielparametern, wie Gesamt-Ultrafiltrationsvolumen, maximale Ultrafiltrationsrate, Behandlungsdauer, mit einem Blutdruckmessgerät (24) und einer gedächtnisgestützten Regeleinrichtung (23) zur Regelung des Blutdrucks eines Patienten, wobei die gedächtnisgestützte Regeleinrichtung zum Ausführen folgender Funktionen nach einer Blutdruckmessung ausgebildet ist:
a) Erfassung und Speicherung des Zeitverlaufs des Blutdrucks während des gegenwärtigen Therapievorganges;
b) Analytische Vergleiche des Zeitverlaufs während des gegenwärtigen Therapievorganges mit Zeitverläufen vorangegangener Therapievorgänge bis zum aktuellen Zeitpunkt des gegenwärtigen Therapievorganges und Auswählen der vorangegangenen Therapievorgänge mit hoher Ähnlichkeit des Blutdruckverlaufs bis zum aktuellen Zeitpunkt;
c) Analysieren der Verläufe des Blutdruckes der ausgewählten vorangegangenen Therapievorgänge in auf den aktuellen Zeitpunkt des gegenwärtigen Therapievorganges nachfolgenden Zeitabschnitten auf Blutdruckabfälle und Selektieren mindestens eines der vorangegangenen Therapievorgänge mit Blutdruckabfall in den nachfolgenden Zeitabschnitten;
d) Vorausschauende Blutdruckregelung zur Prävention von Blutdruckabfällen, wobei der Zeitverlauf des mindestens einen selektierten Therapievorganges als Führungsgröße (FG) der vorausschauenden Blutdruckregelung in den auf den aktuellen Zeitpunkt nachfolgenden Zeitabschnitten verwendet wird.

2. Therapieeinrichtung nach Anspruch 1, **dadurch gekennzeichnet, dass** die Regeleinrichtung derart ausgebildet ist, dass sie die Funktion b) und c) nacheinander wiederholt ausführt.

3. Therapieeinrichtung nach Anspruch 1 oder 2, **dadurch gekennzeichnet, dass** die Regeleinrichtung (23) entscheidet, aus welchen vorangegangenen Therapievorgängen die Führungsgröße der vorausschauenden Blutdruckregelung für den nachfolgenden Zeitabschnitt des gegenwärtigen Therapievorganges übernommen wird.

4. Therapieeinrichtung nach einem der Ansprüche 1-3, **dadurch gekennzeichnet,**
**dass** die Zeitverläufe blutdruckrelevanter Einflussgrößen, wie Ultrafiltrationsrate, Dialysatleitfähigkeit, Infusionsmenge, und die einstellbaren Zielparameter, wie Gesamt-Ultrafiltrationsvolumen, maximale Ultrafiltrationsrate, Behandlungsdauer, jedes Therapievorganges erfasst und gespeichert werden; und
**dass** mindestens einer der Zeitverläufe blutdruckrelevanter Einflussgrößen aus den selektierten vorangegangenen Therapievorgängen als zusätzliche Führungsgröße der vorausschauenden Blutdruckregelung in auf den aktuellen Zeitpunkt des gegenwärtigen Therapievorganges nachfolgenden Zeitabschnitten verwendet wird.

5. Therapieeinrichtung nach einem der Ansprüche 1-4, wobei der Ermittlung der Ähnlichkeit vorangegangener Therapievorgänge mit dem gegenwärtigen Therapievorgang die Zeitabschnitte vom Beginn des Therapievorganges bis zum aktuellen Zeitpunkt und/oder kürzere Zeitabschnitte, z. B. 15 Minuten vor dem aktuellen Zeitpunkt, zugrunde liegen.

6. Therapieeinrichtung nach einem der Ansprüche 1-5, wobei von der gedächtnisgestützten Regeleinrichtung alle oder einige der folgenden Kategorien von Blutdruckwerten oder Blutdruck beeinflussenden Werten in die vorausschauende Blutdruckregelung einbezogen werden:
- Blutdruckwerte, die durch intervallartige Messungen gemäß dem in der Regeleinrichtung implementierten Zeitregime ermittelt werden,
- Blutdruckwerte, die zwecks Reduzierung der Anzahl der Blutdruckmessungen in Form von Leitkurven aus vorangegangenen Therapievorgängen substituiert werden,
- Blutdruckwerte oder Blutdruck beeinflussende Werte, die von Mess- und Auswertegeräten für andere physiologische Größen (zum Beispiel EKG, Relatives Blutvolumen, Hämatokrit, Blutsauerstoffgehalt) automatisch angefordert werden, und
- Blutdruckwerte, die vom medizinischen Personal manuell angefordert werden.

## Claims

1. A therapy device for extracorporeal blood treatment with settable target parameters such as e.g. total ultrafiltration volume, maximal ultrafiltration rate, length of treatment, by use of a blood-pressure measuring device (24) and a memory-supported control means (23) for controlling the blood pressure of a patient, wherein said memory-supported control means is configured to perform the following functions after a blood-pressure measurement:
a) detection and storage of the temporal development of the blood pressure during the actual therapy procedure;
b) analytical comparisons of the temporal development during the actual therapy procedure with temporal developments of preceding therapy procedures up to the actual point of time of the actual therapy procedure, and selection of preceding therapy procedures with high similarity in the blood-pressure development up to the actual point of time;
c) analyzing the developments of the blood pressure of said selected preceding therapy procedures in time periods following the actual point of time of the running therapy procedure for detection of drops in blood pressure, and selecting at least one of the preceding therapy procedures with a drop in blood pressure in the subsequent time periods;
d) prospective blood-pressure control for prevention of drops in blood pressure, wherein the temporal development of said at least one selected therapy procedure is used as a guide value (FG) of said prospective blood-pressure control in the time periods following the actual point of time.

2. The therapy device according to claim 1, **characterized in that** said control device is configured to repeatedly perform said functions b) and c) subsequently to each other.

3. The therapy device according to claim 1 or 2, **characterized in that** said control device (23) is operative to decide from which preceding therapy procedures the guide value of the prospective blood-pressure control is to be taken over for the subsequent time period of the actual therapy procedure.

4. The therapy device according to any one of claims 1-3, **characterized in**
**that** the temporal developments of blood-pressure-relevant influencing variables such as e.g. ultrafiltration rate, dialysate conductivity and infusion quantity, and the settable target parameters such as e.g. total ultrafiltration volume, maximal ultrafiltration rate and length of treatment of each therapy procedure are detected and stored; and
**that** at least one of said temporal developments of blood-pressure-relevant influencing variables from the selected preceding therapy procedures is used as an additional guide value of the prospective blood-pressure control in time periods following the actual point of time of the actual therapy procedure.

5. The therapy device according to any one of claims 1-4, wherein the detection of a similarity of preceding therapy procedures to the actual therapy procedure is based on the time periods from the start of the therapy procedure up to the actual point of time, and/or on shorter time periods, e.g. 15 minutes prior to the actual point of time.

6. The therapy device according to any one of claims 1-5, wherein, by the memory-supported control means, all or some of the following categories of blood-pressure values or blood-pressure-influencing variables are included in the prospective blood-pressure control:
- blood-pressure values which are detected by interval-based measurements according to the time regime implemented in the control means,
- blood-pressure values which are substituted in the form of guide curves from preceding therapy procedures for thus reducing the number of blood-pressure measurements,
- blood-pressure values or blood-pressure-influencing variables which are automatically requested by measurement and analysis devices for other physiological values (e.g. ECG, relative blood volume, hemocrit, blood-oxygen content), and
- blood-pressure values which are requested through manual input by the medical personnel.

## Revendications

1. Dispositif de thérapie pour le traitement de sang extracorporel avec des paramètres de but réglables, comme le volume total de l'ultrafiltration, le taux maximal de l'ultrafiltration, la durée de traitement, comprenant un oscillomètre (24) et un moyen de régulation (23) basé sur mémoire pour réguler la pression artérielle d'un sujet, le moyen régulation basé sur mémoire étant configuré pour exécuter les fonctions suivantes après une mesurage de la pression artérielle:
a) détection et stockage du cours temporel de la pression artérielle pendant le procès de thérapie actuel;
b) comparer analytiquement le cours temporel pendant le procès de thérapie actuel avec des cours temporels des procès de thérapie précédents jusqu'au moment actuel du procès de thérapie actuel, et choisir des procès de thérapie précédents avec un cours de la pression artérielle très semblable jusqu'au moment actuel;
c) analyser les cours de la pression artérielle du procès de thérapie précédents choisis dans des périodes suivant le moment actuel du procès de thérapie actuel pour des pertes de pression artérielle, et sélecter au moins un des procès de thérapie précédents avec une perte de pression artérielle dans le périodes suivantes;
d) réguler la pression artérielle de manière proactive pour la prévention de pertes de pression artérielle, le cours temporel de l'au moins un procès de thérapie sélecté servant comme valeur de pilotage (FG) de la régulation proactive de la pression artérielle dans les périodes suivant le moment actuel.

2. Dispositif de thérapie selon la revendication 1, **caractérisé en ce que** le moyen de régulation est configuré de sorte qu'il exécute les fonctions b) et c) l'une après l'autre d'une manière répétitive.

3. Dispositif de thérapie selon les revendications 1 ou 2, **caractérisé en ce que** le moyen de régulation (23) décide duquel des procès de thérapie précédents le valeur pilotage de la régulation proactive de la pression artérielle sera adopté pour la période suivante du procès de thérapie actuel.

4. Dispositif de thérapie selon l'une quelconque des revendications 1-3, **caractérisé en ce que**
les cours temporels de valeurs d'influence important pour la pression artérielle, comme le taux de l'ultrafiltration, la conductivité de dialysat, le volume d'infusion, et les paramètres de but réglables, comme le volume total de l'ultrafiltration, le taux maximal de l'ultrafiltration, la durée de traitement, de chaque procès de thérapie sont détectés et stockés; et
au moins un des cours temporels de valeurs d'influence important pour la pression artérielle des procès de thérapie précédents sélectés est utilisé comme valeur de pilotage additionnel de la régulation proactive de la pression artérielle pendant des périodes suivant le moment actuel du procès de thérapie actuel.

5. Dispositif de thérapie selon l'une quelconque des revendications 1-4, dans lequel la détermination de la similarité entre des procès de thérapie précédents et le procès de thérapie actuel est basée sur les périodes depuis le commencement du procès de thérapie jusqu'au moment actuel et/ou sur des périodes plus courtes, pour exemple 15 minutes avant le moment actuel.

6. Dispositif de thérapie selon l'une quelconque des revendications 1-5, dans lequel, pour la régulation proactive de la pression artérielle, le moyen de régulation basé sur mémoire tient compte de toutes ou de quelques unes des catégories suivantes de valeurs de pression artérielle ou de valeurs avec influence sur la pression artérielle:
- valeurs de la pression artérielle déterminées par mesurage de type à intervalle correspondant au régime temporel implémenté dans le moyen de régulation,
- valeurs de la pression artérielle substituées des procès de thérapie précédents en forme de directrices afin de réduire le nombre de mesurages de la pression artérielle,
- valeurs de la pression artérielle ou valeurs avec influence sur la pression artérielle, qui sont automatiquement demandées par des appareils de mesurage et d'évaluation pour autres paramètres physiologiques (pour exemple ECG, volume de sang rélatif, hématocrite, teneur en oxygène du sang), et
- valeurs de la pression artérielle manuellement demandées par le personnel médical.
